# EUROPEAN PATENT APPLICATION

(11) **EP 4 599 824 A1**
(43) Date of publication of application: **13.08.2025**
(21) Application number: 25155921.7
(22) Date of filing: 05.02.2025
(51) Int. Cl.: A61K 9/20, A61K 31/496

(54) **A SOLID ORAL PHARMACEUTICAL FORMULATION COMPRISING AVATROMBOPAG**

(30) Priority: 06.02.2024 TR 202401353
(71) Applicant: Sanovel Ilac Sanayi Ve Ticaret Anonim Sirketi, Istanbul (TR)
(72) Inventor: SUNEL, Fatih, Istanbul (TR); ATAK, Fadime Bilgehan, Istanbul (TR); GULER, Tolga, Istanbul (TR)
(74) Representative: Genç Ilhan, Oznur

(57) **Abstract**

The present invention relates to a solid oral pharmaceutical formulation comprising avatrombopag maleate or a pharmaceutically acceptable salt, amorphous or crystalline form thereof and at least one filler, wherein the filler is lactose anhydrous. The present invention also relates to a simple, rapid, cost-effective, time-saving and industrially suitable process for preparing the solid oral pharmaceutical formulation.

## Description

### Field of Invention

The present invention relates to a solid oral pharmaceutical formulation comprising avatrombopag maleate or a pharmaceutically acceptable salt, amorphous or crystalline form thereof and at least one filler, wherein the filler is lactose anhydrous. The present invention also relates to a simple, rapid, cost-effective, time-saving and industrially suitable process for preparing the solid oral pharmaceutical formulation.

### Background of the Invention

Avatrombopag, sold under the brand name Doptelet, is a medication that used for certain conditions that lead to thrombocytopenia (low platelets) such as thrombocytopenia associated with chronic liver disease in adults who are to undergo a planned medical or dental procedure. The molecular formula of avatrombopag maleate is C₂₉H₃₄Cl₂N₆O₃S₂·C₄H₄O₄, and its structural formula(I) is as follows:

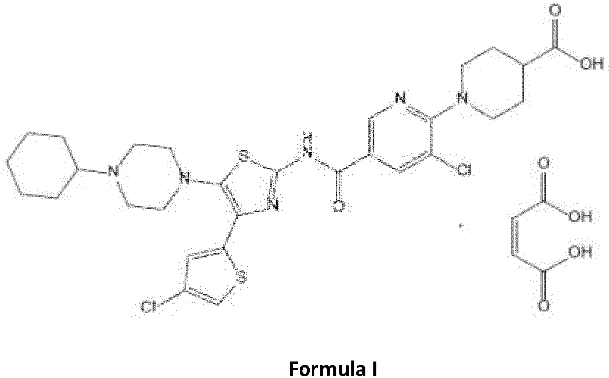

Avatrombopag is a white to off white powder with a nonchiral structure. The compound is practically insoluble in water and is nonhygroscopic. However, avatrombopag maleate is almost insoluble in water and 0.1M hydrochloric acid and is almost insoluble in the pH range of all systems in the body.

Avatrombopag maleate is an oral, small molecule thrombopoietin (TPO) receptor (c-Mpl) agonist, which can simulate the biological effect of TPO, stimulate platelet production, improve platelet count, belonging to the clinical urgent need medicine.

Avatrombopag generally used in solid form in the formulation, drug polymorphism is one of the important factors influencing the quality of medicine and clinical curative effect, therefore, has very important meaning to research of the crystal form.

The patent RU2769863C1 disclose the invention relates to the field of medicine, namely, to hematology, and is intended for treating thrombocytopenia.

The patent CN114010638A disclose the invention belongs to the field of medical technology and relates to therapeutic drugs for thrombocytopenia associated with chronic liver disease (CLD), and in particular to a stable preparation composition of avatrombopag maleate and its prepared tablets or capsules.

There are many patent applications for avatrombopag in the prior art. Different ways have been tried to overcome the low solubility of the active substance, such as the use of micronized active substances or processes consisting of multiple steps. There is still a need for a new and simple formulation and process. With this present invention the desired dissolution profile and stability of the solid oral pharmaceutical formulation comprising avatrombopag maleate will be achieved.

In this invention, we found that the use of avatrombopag maleate with lactose anhydrous overcomes the above-mentioned problems and provides additional advantages over the prior art.

### Detailed description of the Invention

The main object of the present invention is a solid oral pharmaceutical formulation comprising avatrombopag maleate having the desired solubility and stability.

Another object of the present invention is a solid oral pharmaceutical formulation comprising avatrombopag maleate which has the desired content uniformity, flowability and compressibility.

Another object of the present invention is to provide a preparation process of a solid oral pharmaceutical formulation comprising avatrombopag maleate which is simple and cost-effective process.

It is known in the prior art that avatrombopag maleate is not hygroscopic and does not dissolve in water. A new formulation is still needed for this. We have found that the use of avatrombopag in combination with lactose anhydrous results in a formulation with the desired stability and dissolution profile.

Especially in our studies, we tried different forms of lactose as fillers. Our experiments showed that compressibility problems show when lactose monohydrate was used. However, especially the use of lactose anhydrous give the best results for compressibility. So, we achieved the desired stability and dissolution profile and overcame the compressibility problem.

According to one embodiment of the invention, a solid oral pharmaceutical formulation comprising avatrombopag maleate or a pharmaceutically acceptable salt, amorphous and crystalline form thereof and at least one filler, wherein the filler is lactose anhydrous.

According to one embodiment of this invention, the amount of lactose anhydrous is between 50.0% and 65.0% by weight of the solid oral pharmaceutical formulation.

Avatrombopag maleate is a crystal medicine with low solubility and low permeability, solubility is small, bioavailability is low, it is almost insoluble in the range of pH 1-9. Therefore, the dissolution is the speed limiting step absorbed in the preparation body.

According to one embodiment of this invention, avatrombopag maleate is present in the form of crystalline form A or crystalline form B. The use of avatrombopag as crystal form A or form B provided rapid absorption and improved solubility.

According to this embodiment of the invention, the amount of avatrombopag maleate is between 5.0% and 25.0% by weight of the solid oral pharmaceutical formulation. Preferably, the amount of avatrombopag maleate is between 8.0% and 15.0% by weight of the solid oral pharmaceutical formulation.

According to an embodiment of the present invention, the solid oral pharmaceutical formulation further comprises at least one pharmaceutically acceptable excipient which is selected from the group comprising fillers, disintegrants, lubricants or a mixture thereof.

According to one embodiment of this invention, the formulation further comprises at least one filler. Suitable fillers are selected from the group comprising microcrystalline cellulose, ammonium alginate, calcium carbonate, calcium phosphate, calcium phosphate dehydrate, neutral pellets, calcium sulfate, cellulose, cellulose acetate, compressible sugar, dextrates, dextrin, dextrose, ethylcellulose, fructose, glyceryl palmitostearate, lactitol, mannitol, polymethacrylates, simethicone, sodium alginate, sodium chloride, sorbitol, sucrose, sugar spheres, sulfobutylether beta-cyclodextrin, tragacanth, trehalose, xylitol or mixtures thereof.

According to one embodiment of the present invention, the filler is microcrystalline cellulose.

According to one embodiment of the present invention, the amount of microcrystalline cellulose is between 10.0% and 30.0% by weight of the solid oral pharmaceutical formulation.

According to this embodiment of the present invention, the weight ratio of lactose anhydrous to microcrystalline cellulose is between 1.5 and 6.5.

Suitable disintegrants are selected from the group comprising croscarmellose sodium, sodium starch glycolate, crospovidone, low-substituted hydroxypropyl cellulose, sodium carboxymethyl cellulose, calcium carboxymethyl cellulose, carboxymethyl cellulose, docusate sodium, guar gum, polyacrylate potassium, sodium alginate, corn starch, alginic acid, alginates, ion-exchange resins, magnesium aluminum silica, sodium dodecyl sulphate, poloxamer, sodium glycine carbonate, sodium lauryl sulphate or mixtures thereof.

According to one embodiment of the present invention, the amount of disintegrant is between 1.0% and 9.0% by weight of the solid oral pharmaceutical formulation.

According to one embodiment of the present invention, the disintegrant is croscarmellose sodium or sodium starch glycolate.

Suitable lubricants are selected from the group comprising from magnesium stearate, talc, calcium stearate, zinc stearate, waxes, boric acid, hydrogenated vegetable oil, sodium chlorate, magnesium lauryl sulfate, sodium oleate, sodium acetate, sodium benzoate, polyethylene glycol, stearic acid, fatty acid, fumaric acid, glyceryl palmito sulphate, sodium stearyl fumarate, sodium lauryl sulphate or mixtures thereof.

According to one embodiment of the present invention, the amount of lubricant is between 0.01% and 8.0% by weight of the solid oral pharmaceutical formulation.

According to one embodiment of the present invention, the lubricant is magnesium stearate.

According to one embodiment of the present invention, the solid oral pharmaceutical formulation is in the form of tablets, capsules, strips, powders, pastilles, sachets, effervescent compositions, pills, coated bead systems, granules, microspheres, dragees, films, orally administrable films, solids.

Preferably, the solid oral pharmaceutical formulation is in the form of tablets or capsule.

In the present invention, the solid oral pharmaceutical formulation is tablet, and it comprises film coating to protect the composition against the moisture and light to maintain the stability. Suitable film coating agents are selected from the group comprising opadry yellow, polyvinyl alcohol (PVA), titanium dioxide, macrogol (PEG 3350), Indigo carmine aluminum lake, carnauba wax, hydroxypropyl methylcellulose, polyethylene glycol (PEG), polyvinyl alcohol-polyethylene glycol copolymers, lecithin, polyvinylpyrrolidone-vinyl acetate copolymer (PVP-VA), pigments, dyes, iron oxide or a mixture thereof. Preferably film coating agent is Opadry yellow.

According to this embodiment of the present invention, the amount of film coating agent is between 1.0% and 8.0% by weight of the total formulation.

According to this embodiment of the present invention, the solid oral pharmaceutical formulation comprises;
- Avatrombopag maleate form A or B
- Microcrystalline cellulose
- Lactose anhydrous
- Croscarmellose sodium or sodium starch glycolate,
- Colloidal silicon dioxide
- Magnesium stearate

According to one embodiment of the present invention, the solid oral pharmaceutical formulation comprising avatrombopag maleate or a pharmaceutically acceptable salt, amorphous and crystalline form thereof is obtained by direct compression process. In direct compression, the solid pharmaceutical compositions ingredients are not exposed to moisture, solvents and heat. Thus, in this invention, direct compression is used to process moisture, solvent and/or heat sensitive avatrombopag. In addition, this process provides the desired compressibility and flowability without loss of active substance. The desired dissolution profile and stability of the solid oral pharmaceutical formulation is obtained and it has a simple preparation process suitable for industrial production.

According to another embodiment of the present invention, a process for preparing the solid oral pharmaceutical formulation comprises the following steps;
- Weighing of the avatrombopag maleate form A or form B and all excipients,
- Mixing fillers, disintegrant, glidant and active substance,
- Sieving the mixture,
- Adding lubricant to the mixture and mixing,
- Compressing the homogeneous mixture obtained in such a way that each tablet is of the specified specifications,
- Coating the core tablet with film coating. ***Example 1; Tablet formulation***

| **Ingredients** | **% by weight of the total tablet** | **% by weight of the total tablet** |
|---|---|---|
| Avatrombopag maleate form A or form B | 11.3 | 5-25 |
| Microcrystalline cellulose | 20 | 10-30 |
| Lactose anhydrous | 58.1 | 50-65 |
| Croscarmellose sodium | 2.9 | 1-10 |
| Colloidal silicon dioxide | 2.9 | 1-10 |
| Magnesium stearate | 1 | 0.01-8 |
| **Tablet core weight** | 96.2 | 92-98 |
| Opadry yellow | 3.8 | 1-8 |
| **Total tablet** | 100 | 100 |

### Example 2; Tablet formulation

| **Ingredients** | **% by weight of the total tablet** | **% by weight of the total tablet** |
|---|---|---|
| Avatrombopag maleate form A or form B | 11.3 | 5-25 |
| Microcrystalline cellulose | 20 | 10-30 |
| Lactose anhydrous | 58.1 | 50-65 |
| Sodium starch glycolate | 2.9 | 1-10 |
| Colloidal silicon dioxide | 2.9 | 1-10 |
| Magnesium stearate | 1 | 0.01-8 |
| **Tablet core weight** | 96.2 | 92-98 |
| Opadry yellow | 3.8 | 1-8 |
| **Total tablet** | 100 | 100 |

A process for example 1-2;
- Weighing of the active substance and all excipients,
- Mixing lactose anhydrous, microcrystalline cellulose, croscarmellose sodium or sodium starch glycolate, colloidal silicon dioxide and avatrombopag maleate form A or form B,
- Sieving the mixture,
- Adding magnesium stearate to the mixture and mixing,
- Pressing the homogeneous mixture obtained in such a way that each tablet is of the specified specifications,
- Film coating of pressed tablets.

## Claims

1. A solid oral pharmaceutical formulation comprising avatrombopag maleate or a pharmaceutically acceptable salt, amorphous and crystalline form thereof and at least one filler, wherein the filler is lactose anhydrous.

2. The solid oral pharmaceutical formulation according to claim 1, wherein amount of lactose anhydrous is between 50.0% and 65.0% by weight of the solid oral pharmaceutical formulation.

3. The solid oral pharmaceutical formulation according to claim 1, wherein avatrombopag maleate is present in the form of crystalline form A or crystalline form B.

4. The solid oral pharmaceutical formulation according to claim 1 or 3, wherein amount of avatrombopag maleate is between 5.0% and 25.0% by weight of the solid oral pharmaceutical formulation.

5. The solid oral pharmaceutical formulation according to claim 1, wherein further comprises at least one pharmaceutically acceptable excipient which is selected from the group comprising fillers, disintegrants, lubricants or a mixture thereof.

6. The solid oral pharmaceutical formulation according to claim 5, wherein fillers are selected from the group comprising microcrystalline cellulose, ammonium alginate, calcium carbonate, calcium phosphate, calcium phosphate dehydrate, neutral pellets, calcium sulfate, cellulose, cellulose acetate, compressible sugar, dextrates, dextrin, dextrose, ethylcellulose, fructose, glyceryl palmitostearate, lactitol, mannitol, polymethacrylates, simethicone, sodium alginate, sodium chloride, sorbitol, sucrose, sugar spheres, sulfobutylether beta-cyclodextrin, tragacanth, trehalose, xylitol or mixtures thereof.

7. The solid oral pharmaceutical formulation according to claim 6, wherein another filler is microcrystalline cellulose.

8. The solid oral pharmaceutical formulation according to claim 1 or 7, wherein weight ratio of lactose anhydrous to microcrystalline cellulose is between 1.5 and 6.5.

9. The solid oral pharmaceutical formulation according to claim 5, wherein disintegrants are selected from the group comprising croscarmellose sodium, sodium starch glycolate, crospovidone, low-substituted hydroxypropyl cellulose, sodium carboxymethyl cellulose, calcium carboxymethyl cellulose, carboxymethyl cellulose, docusate sodium, guar gum, polyacrylate potassium, sodium alginate, corn starch, alginic acid, alginates, ion-exchange resins, magnesium aluminum silica, sodium dodecyl sulphate, poloxamer, sodium glycine carbonate, sodium lauryl sulphate or mixtures thereof.

10. The solid oral pharmaceutical formulation according to claim 9, wherein disintegrant is croscarmellose sodium or sodium starch glycolate.

11. The solid oral pharmaceutical formulation according to claim 5, wherein lubricants are selected from the group comprising from magnesium stearate, talc, calcium stearate, zinc stearate, waxes, boric acid, hydrogenated vegetable oil, sodium chlorate, magnesium lauryl sulfate, sodium oleate, sodium acetate, sodium benzoate, polyethylene glycol, stearic acid, fatty acid, fumaric acid, glyceryl palmito sulphate, sodium stearyl fumarate, sodium lauryl sulphate or mixtures thereof.

12. The solid oral pharmaceutical formulation according to claim 11, wherein lubricant is magnesium stearate.

13. The solid oral pharmaceutical formulation according to claim 1 or 5, wherein formulation comprises;
• Avatrombopag maleate form A or B
• Microcrystalline cellulose
• Lactose anhydrous
• Croscarmellose sodium or sodium starch glycolate,
• Colloidal silicon dioxide
• Magnesium stearate

14. The solid oral pharmaceutical formulation according to claim 1, wherein avatrombopag maleate or a pharmaceutically acceptable salt, amorphous and crystalline form thereof is obtained by direct compression process.
